# EUROPÄISCHE PATENTSCHRIFT

(11) **EP 1 644 040 B1**
(45) Veröffentlichungstag und Bekanntmachung des Hinweises auf die Patenterteilung: **09.12.2009**
(21) Anmeldenummer: 04730971.1
(22) Anmeldetag: 04.05.2004
(51) Int. Cl.: A61K 45/06, A61P 31/20

(54) **VERWENDUNG VON KOMBINATIONEN AUS HEMMERN DER REVERSEN TRANSKRIPTASE UND HEMMERN DER VIRUSCODIERTEN DNA-POLYMERASE**
USE OF COMBINATIONS OF REVERSE TRANSCRIPTASE INHIBITORS AND VIRUS CODED DNA POLYMERASE INHIBITORS
UTILISATION DE COMBINAISONS D'INHIBITEURS DE LA TRANSCRIPTASE INVERSE ET D'INHIBITEURS DE L'ADN-POLYMERASE CODEE PAR VIRUS

(30) Priorität: 05.05.2003 DE 10321905
(43) Veröffentlichungstag der Anmeldung: 12.04.2006
(73) Patentinhaber: Eberhard-Karls-Universität Tübingen Universitätsklinikum, 72076 Tübingen (DE)
(72) Erfinder: HAMPRECHT, Klaus, 72074 Tübingen (DE); SCHOTT, Herbert, 72076 Tübingen (DE); MIKELER, Elfiede, 72076 Tübingen (DE); JAHN, Gerhard, 72108 Rottenburg (DE)
(74) Vertreter: Laufer, Gabriele
(86) Internationale Anmeldenummer: PCT/EP2004/004693
(87) Internationale Veröffentlichungsnummer: WO 2004/098640

(56) Entgegenhaltungen:
- WO-A-99/41268
- US-A- 5 686 428
- DATABASE BIOSIS [Online] BIOSCIENCES INFORMATION SERVICE, PHILADELPHIA, PA, US; 1996, MULATO A S ET AL: "Anti-HCMV activity of cidofovir in combination with antiviral compounds and immunosuppressive agents: In-vitro analyses" XP002293963 Database accession no. PREV199699131041 & ANTIVIRAL CHEMISTRY AND CHEMOTHERAPY, Bd. 7, Nr. 4, 1996, Seiten 203-208, ISSN: 0956-3202
- DENG DAVID X L ET AL: "In vivo antihepadnaviral activities of combination of famciclovir, lamivudine, and foscarnet" ANTIVIRAL RESEARCH, Bd. 50, Nr. 1, April 2001 (2001-04), Seite A80, XP002293960 & FOURTEENTH INTERNATIONAL CONFERENCE ON ANTIVIRAL RESEARCH; SEATTLE, WASHINGTON, USA; APRIL 08-12, 2001 ISSN: 0166-3542
- FREITAS VICKI R ET AL: "Efficacy of ganciclovir in combination with zidovudine against cytomegalovirus in vitro and in vivo" ANTIVIRAL RESEARCH, Bd. 21, Nr. 4, 1993, Seiten 301-315, XP002293961 ISSN: 0166-3542
- GERARD LAURENCE ET AL: "Pharmacology and clinical use of foscarnet" INTERNATIONAL JOURNAL OF ANTIMICROBIAL AGENTS, Bd. 5, Nr. 4, 1995, Seiten 209-217, XP002293962 ISSN: 0924-8579

## Beschreibung

Die vorliegende Erfindung betrifft die Verwendung von antiviralen Substanzen zur Behandlung von Viruserkrankungen, die durch DNA-Viren ausgelöst werden.

Die Forschung nach antiviralen Wirkstoffen wird insbesondere durch den obligaten Zellparasitismus der Viren erschwert. Da Viren zelluläre Enzyme und Stoffwechselwege nutzen, beschränkt sich ihre genetische Information auf ein Minimum. Für die antivirale Chemotherapie bedeutet dies, dass eine Hemmung der Viren meist mit einer erheblichen Zelltoxizität verbunden ist.

Demnach werden derzeit nur einige der Schritte der Virusvermehrung als Angriffspunkte für antivirale Chemotherapeutika genutzt. Ein wichtiger Angriffspunkt ist die Hemmung der Nukleinsäuresynthese. Hier werden insbesondere Nukleosidanaloge eingesetzt, die anstelle der physiologischen Bausteine von viralen Polymerasen als Substrat angenommen werden. Durch den Einbau der Nukleosidanalogen in die wachsende DNA-Kette kommt es zur Enzymblockade und somit zum Abbruch der DNA-Synthese.

Die Gefahr, an viralen Infektionen zu erkranken, ist insbesondere bei immunsupprimierten Patienten groß, also bspw. nach Transplantation von soliden Organen, Knochenmark oder peripheren Stammzellen sowie bei Patienten mit AIDS. Neben dem humanen Immundefizienz-Virus (HIV) spielen insbesondere Mitglieder der Familie der Herpesviridae bei Infektionserkrankungen eine große Rolle. Zu dieser Gruppe zählen bspw. das Herpes simplex Virus (HSV), das Varicella zoster Virus (VZV), das Epstein-Barr Virus (EBV) und das humane Cytomegalovirus (HCMV).

Derzeit stehen drei antivirale Wirksubstanzen für einen Einsatz bei Erkrankungen zur Verfügung, die durch HCMV hervorgerufen werden: Ganciclovir (GCV) und Ganciclovir-Derivate, Foscarnet (Natrium-Phosphonoameisensäure, PFA) sowie Cidofovir (CDV).

Ganciclovir ist ein acyclisches Guanosinanaloges, Cidofovir ein acyclisches dCMP-Analoges. Die Substanzen unterbinden die virale DNA-Synthese letztendlich durch Inhibition der DNA-Polymerase. Um dieses Enzym zu inhibieren, müssen Ganciclovir und Cidofovir jeweils zum Triphosphat phosphoryliert werden. Der Einbau von GCV-TP und CDV-TP in den neu entstehenden viralen DNA-Strang führt zum Kettenabbruch der viralen DNA-Replikation, wobei nur Ganciclovir durch ein viruscodiertes Enzym monophosphoryliert werden muss.

Foscarnet hingegen blockiert direkt die Pyrophosphatbindungsstelle der viralen DNA-Polymerase und inhibiert somit die Abspaltung von Pyrophosphat aus dNTPs.

Nachteilig bei der Verwendung der genannten Verbindungen ist jedoch, dass durch einen verstärkten Einsatz dieser DNA-Polymerasehemmer sowohl *in vitro* als auch *in vivo* die Bildung einer Vielzahl von Wirkstoff-resistenten Virusstämmen hervorgerufen wird. Molekularbiologische Grundlage der Resistenzen sind meist Mutationen, die auf die Verwendung der genannten Substanzen hin in den Targetenzymen, also bspw. der DNA-Polymerase, entstehen. Die Mutationen sind dabei gewöhnlich charakteristisch für die einzelnen Klassen der antiviralen Verbindungen, oder sogar der einzelnen Verbindungen.

So ist z.B. das HCMV-codierte Enzym Phosphotransferase (UL-97) in Ganciclovir-resistenten HCMV-Stämmen mutiert. Dieses Enzym ist am ersten Phosphorylierungsschritt von Ganciclovir beteiligt. Mutanten, die gegenüber Cidofovir und Foscarnet resistent sind, weisen meist zusätzlich Mutationen im viralen DNA-Polymerasegen auf (für eine Übersicht siehe bspw. Erice, "Resistance of human cytomegalovirus to antiviral drugs", Clin. Microbiol. Rev. 1999, 12:286-297).

Beim Nachweis einer Ganciclovir-resistenten HCMV-Infektion wird derzeit als Alternativmedikation Foscarnet eingesetzt. Nachteilig an dieser Vorgehensweise ist jedoch, dass eine relativ hohe Wirkungsdosis an Foscarnet nötig ist, um eine effiziente antivirale Wirkung zu erzielen. Eine hohe Wirkungsdosis wiederum zieht jedoch toxische Nebenwirkungen nach sich.

Besonders kritisch ist die Situation bei der Manifestation einer Multiresistenz gegen Ganciclovir, Foscarnet und Cidofovir, bspw. bei HCMV-Infektionen.

Neben der Ausbildung von Resistenzen spielt ferner die Toxizität der Chemotherapeutika eine große Rolle für deren oftmals beschränkten Einsatz bei viralen Erkrankungen.

So verursacht die Gabe von Ganciclovir insbesondere eine Veränderung der Anzahl der weißen Blutkörperchen und häufig auch der Thrombozyten. Bei einer Verabreichung über längere Zeit kann auch eine Anämie auftreten. Die Verabreichung von Ganciclovir in voller Dosis in Verbindung mit anderen myelotoxischen Pharmaka kann wegen der hämatologischen Toxizität lebensbedrohlich sein (siehe bspw. Crumpacker, "Ganciclovir", N. Engl. J. Med. 1996, 335:721-731).

Cidofovir besitzt eine dosisabhängige Nephrotoxizität. Sie wird durch ein Ungleichgewicht zwischen schneller Aufnahme in die proximalen Tubuluszellen und dem langsameren Efflux in den Urin hervorgerufen, was zur Akkumulation in den Nieren führt.

Foscarnet besitzt eine erhebliche renale und metabolische Toxizität. Die Nephrotoxozität basiert auf der direkten Schädigung der Nierentubuli.

Der vorliegenden Erfindung liegt daher die Aufgabe zugrunde, Substanzen zur Herstellung eines antiviralen Arzneimittels bereitzustellen, welches vorzugsweise bei niedriger Dosierung und weiter vorzugsweise auch bei resistenten und multiresistenten Virusstämmen eine hohe Wirksamkeit bietet.

Erfindungsgemäß wird die Aufgabe gelöst durch die Verwendung nach Anspruch 1.

Die der Erfindung zugrunde liegende Aufgabe wird auf diese Weise vollkommen gelöst.

Die Verbindungen können dabei als Mischung oder als sogenanntes Kombinationspräparat vertrieben werden, bei dem in einer Umverpackung die Verbindungen in getrennten Verpackungseinheiten vorliegen. Es ist aber auch möglich, beide Verbindungsklassen in getrennten Verpackungen zu vertreiben und jeweils in dem Beipackzettel darauf hinzuweisen, dass die Verbindung in Kombination mit der jeweils anderen Verbindung zur Behandlung von Infektionen von DNA-Viren eingesetzt werden kann. Der RTI und der DNA-Polymerase-Hemmer können dabei selbst in einer chemischen Verbindung oder einer Mischung mit anderen Verbindungen vorliegen.

Die Erfinder konnten in eigenen Versuchen zeigen, dass der kombinierte Einsatz von verschiedenen RTI mit einem DNA-Polymerase-Hemmer eine synergistische Virus-hemmende Wirkung gegenüber verschiedenen HCMV-Stämmen zeigten.

Diese Ergebnisse sind überraschend und waren nach dem im Stand der Technik vorliegenden Kenntnissen nicht zu erwarten, wie nachstehend erläutert werden wird.

Die reverse Transkriptase stellt ein Enzym dar, welches Retroviren zur Vermehrung ihrer RNA benötigen. Reverse-Transkriptase-Inhibitoren (RTI) zeigen demnach erwartungsgemäß keine Wirkung gegenüber DNA-Viren. Diese im Stand der Technik bekannte Erkenntnis konnten die Erfinder auch in eigenen Versuchen bestätigen.

Jedoch konnte durch den direkten Vergleich der Wirkung von DNA-Polymerase-Hemmern alleine mit der Wirkung von Kombinationen aus DNA-Polymerase-Hemmern und Reverse-Transkriptase-Inhibitoren bei letzteren völlig unerwartet ein synergistischer Effekt beobachtet werden. Danach war im Vergleich zur Einzelverwendung die Wirkung der Kombinationen gegenüber DNA-Viren bedeutend verbessert. Dies äußerte sich nicht zuletzt darin, dass bei Verwendung einer Kombination beider Substanz-Klassen jeweils geringere Mengen für eine optimale Wirkung eingesetzt werden konnten, als dies bei der Verwendung einer Einzelsubstanz der Fall war.

Entgegen den Erkenntnissen der Wissenschaft konnte somit eine synergistische Wirkung von Substanzen nachgewiesen werden, deren kombinierte Verwendung bei Erkrankungen, die durch DNA-Viren hervorgerufen werden, einen völlig neuen Therapieansatz darstellt. Denn zum einen benötigen DNA-Viren das Targetenzym der RTI nicht, so dass auch eine Verstärkung der Wirkung der - im Stand der Technik bereits eingesetzten - DNA-Polymerase-Hemmer nicht erwartet war. Andererseits konnte darüber hinaus gezeigt werden, dass es durch diese synergistische Wirkung möglich ist, in der Chemotherapie geringere Mengen als üblich an den einzusetzenden Substanzen verwenden.

Damit stehen aber völlig neue antivirale Wirkstoffkombinationen aus an sich bekannten RTI und an sich bekannten DNA-Polymerase-Hemmern zur Verfügung, so dass die jeweils möglichen Nebenwirkungen für den einzelnen Patienten minimiert werden können.

Erfindungsgemäß werden nucleosidanaloge Hemmer der reversen Transkriptase eingesetzt, die aus der Gruppe umfassend 2',3'-Didehydro-2',3'-didesoxy-thymidin (d4T), (-)-β-L-3'-Thia-2',3'-didesoxycytidin (3TC) und 2',3'-Didesoxycytidin (ddC) ausgewählt sind.

Die Erfinder konnten in eigenen Versuchen zeigen, dass diese reverse-Transkriptase-Hemmer in Kombination mit einem DNA-Polymerase-Hemmer im Vergleich mit den DNA-Polymerase-Hemmern alleine eine verstärkte Virus-hemmende Wirkung gegenüber verschiedenen HCMV-Stämmen aufwiesen.

Diese nukleosidanalogen, bzw. nukleosidalen RTI werden gängigerweise als NRTI bezeichnet. Diese Substanzklasse sieht den Desoxynukleotiden, also den "echten" Bausteinen der DNA, sehr ähnlich. Baut die reverse Transkriptase jedoch NRTI in die entstehende komplementäre Virus-DNA (cDNA) ein, so wird sie geblockt und die Vermehrung des Virus ist gestoppt.

AZT (Handelsname: Zidovudin), ddI (Handelsname: Didanosin), ddC (Handelsname: Zalcitabin), d4T (Handelsname: Stavudin) und 3TC (Handelsname: Lamivudin) sind NRTI, die durch zelluläre Enzyme in die korrespondierenden 5'-Triphosphat-Derivate umgewandelt werden müssen, bevor sie die Virus-codierte reverse Transkriptase inhibieren können. Die Substanzen interagieren mit der Substratbindungsstelle der reversen Transkriptase und hemmen die Virus-Replikation zum einen durch kompetitive Inhibierung des Einbaus der natürlichen dNTP-Substrate und andererseits gerade durch ihren Einbau in die wachsende virale DNA-Kette.

Der Einbau von NRTI in die virale DNA führt notwendigerweise zu einem Kettenabbruch, da die NRTI keine 3'-Hydroxylgruppe im Zuckeranteil besitzen, welche für eine weitere Verlängerung der DNA-Kette notwendig wäre.

Die genannten NRTI sind im Stand der Technik hinreichend beschrieben und werden bei der HIV-Therapie eingesetzt. Sie können vorliegend in freier und/oder chemisch derivatisierter Form eingesetzt werden.

Daneben haben Kong et al., "Synergistic inhibition of human immunodeficiency virus type 1 replication in vitro by two-drug and three-drug combinations of 3'-azido-3'-deoxythymidine, phosphonoformate, and 2',3'-dideoxythymidine", Antimicrob. Agents Chemother. 1991, 35(10):2003-2011, zwar einen synergisitschen Effekt von AZT, PFA und ddT gegenüber dem Retrovirus HIV gezeigt. Dies ist jedoch naheliegend, da die Substanzen AZT und ddT Hemmer des Retrovirus-spezifischen Enzyms reverse Transkriptase sind. Die Verwendung von den genannten Kombinationen bei DNA-Viren ist gerade durch die reverse Transkriptase-Spezifität der Hemmer nicht nahegelegt.

Allgemein werden derzeit im Rahmen erfolgreich verlaufender HIV-Therapien (HAART: highly active antiretroviral therapy) Kombinationen von Nukleosidanalogen- oder Nukleotidanalogen-Reverse Transkriptase-Hemmer, nicht-nukleosidanaloge reverse-Transkriptase-Hemmer sowie Proteaseinhibitoren (PI) eingesetzt (s. beispielsweise Squires, "An introduction to nucleoside and nucleotide analogues", Antivir. Ther. 2001; 6(3):1-14; Joly et al., "NNRTI plus PI combinations in the perspective of nucleoside-sparing or nucleoside-failing antiretroviral regimens", AIDS Rev. 2002; 4:18-39; Menendez-Arias, "Targeting HIV: Antiretroviral therapy and development of drug resistance", Trends Pharmacol. Sci. 2002; 23:381-388; De Clerq, "The Role of nonnucleoside reverse transcriptase inhibitors (NNRTIs) in the therapy of HIV-1 infections", Antiviral Res. 1998; 38:153-179).

Obgleich Kombinationen von insbesondere verschiedenen RTI im Fachgebiet zur Behandlung von HIV-Infektionen bekannt sind, ist der Einsatz verschiedener RTI und DNA-Polymerase-Hemmer zur Behandlung von Erkrankungen, die durch DNA-Viren hervorgerufen werden, im Stand der Technik bislang jedoch weder beschrieben noch nahegelegt.

Erfindungsgemäß wird als Hemmer der viruscodierten DNA-Polymerase Foscarnet (PFA) engesetzt.

Die genannten DNA-Polymerase-Hemmer sind im Stand der Technik hinreichend beschrieben. GCV und CDV werden insbesondere bei der Behandlung von durch HCMV ausgelöste Krankheiten eingesetzt, wohingegen PFA auch bei der Behandlung von Viruskrankheiten verwendet wird, die durch HIV, HSV oder VZV ausgelöst werden.

Da eine verstärkte Verwendung von GCV, PFA und CDV oftmals zur Ausbildung von resistenten Viren-Stämmen führt und die genannten Substanzen insbesondere bei einem Einsatz in höheren Dosen toxisch sind, bietet die erfindungsgemäße Verwendung von Kombinationen aus diesen Substanzen mit RTI den Vorteil, dass auch geringere Dosen dieser DNA-Polymerase-Hemmer eingesetzt werden können. Dies wird durch die synergistische Wirkung beider Substanz-Klassen - auch bei einem Einsatz geringerer Dosen - ermöglicht. Das Risiko toxischer Nebenwirkungen kann demnach deutlich gesenkt werden.

Im Rahmen der erfindungsgemäßen Verwendung versteht sich, dass neben der Möglichkeit der Kombination von lediglich einem RTI und einem viruscodierten DNA-Polymerase-Hemmer auch ein Einsatz von zwei oder mehreren Verbindungen beider Substanz-Klassen als kombinatorische Verwendung zur Herstellung eines Arzneimittels zur Behandlung von durch DNA-Viren ausgelöste Viruserkrankungen möglich ist. In der kombinatorischen Verwendung mit 3 und mehr Verbindungen können 2 oder mehr Hemmer in einer Kombination chemisch miteinander verbunden sein. Die chemische Bindung kann bspw. wie in der Patentanmeldung WO 00/34298 gezeigt erfolgen. Dabei kann bspw. die Dosismenge, die normalerweise für einen Vertreter der RTI in der Kombination eingesetzt wird, deutlich reduziert werden, wobei die Dosismenge des DNA-Polymerase-Hemmers gleich bleibt.

Aus dem Stand der Technik ist bekannt, dass bspw. PFA eine virusinhibierende Wirkung bei HBV- und HCV-Infektionen besitzt. Wegen der vergleichbaren Wirkmechanismen gehen die Erfinder davon aus, dass die Kombinationen auch bei den genannten Viren wirken. Danach kann durch die Kombination von bspw. PFA mit einem RTI die virusinhibierende Wirkung weiter verstärkt werden.

Die Kombinationen werden zur Herstellung eines Arzneimittels zur Behandlung von Infektionskrankheiten eingesetzt, die durch das humane Cytomegalovirus (HCMV) hervorgerufen werden. Menschen mit besonderem Risiko für schwerwiegende HCMV-Erkrankungen sind HIV-infizierte Patienten, bei denen die Funktion des Immunsystems gestört ist. Patienten, die nach einer Transplantation solider Organe medikamentöse Immunsuppression benötigen, sind insbesondere in den ersten drei Monaten ebenfalls gefährdet. Ferner haben Empfänger von Knochenmark- und Stammzelltransplantationen ein hohes Risiko, eine HCMV-Erkrankung zu erleiden, bis das mit der Transplantation übertragene Immunsystem seine volle Funktionsfähigkeit aufgenommen hat.

Insbesondere Neuinfektionen mit HCMV oder eine Reaktivierung kann bei Patienten mit einem supprimierten Immunsystem zu schwersten generalisierten Infektionen mit letalem Ausgang führen. Daher ist die Verwendung der Kombinationen beispielsweise bei Aids-Patienten mit einer HCMV-Infektion vorteilhaft, da somit gleichzeitig zwei Erreger angegriffen werden können.

Ein *in vitro*-Synergismus von Ganciclovir und Foscarnet, also zwei DNA-Polymerase-Hemmern, gegenüber der HCMV-Replikation ist bereits seit längerem bekannt (s. beispielsweise Manischewitz et al., "Synergistic Effect of Ganciclovir and Foscarnet on Cytomegalovires Replication in vitro", Antimicrob. Agents Chemother. 1990; 34: 373 - 375). Über den *in vitro*-Synergismus von NRTI und PFA gegenüber HCMV-Wildstämmen und GCV-resistenten HCMV UL97-Mutanten war bisher jedoch nichts bekannt.

Ferner ist bevorzugt, wenn die Kombination zur Herstellung eines Arzneimittels zur Behandlung von Viruserkrankungen eingesetzt werden, die durch Ganciclovir-sensitive und/oder -resistente humane Cytomegalovirus-Stämme hervorgerufen werden.

Weiterhin ist bevorzugt, wenn die Kombination zur Herstellung eines Arzneimittels zur Behandlung von Viruserkrankungen eingesetzt werden, die durch multi-resistente humane Cytomegalovirus-Stämme hervorgerufen werden.

Durch die erfindungsgemäßen Kombinationen werden neue Therapiemöglichkeiten von HCMV-Infektionen bereitgestellt. Demnach ist es erstmals möglich, GCV-resistente und multi-resistente HCMV-Infektionen effektiv antiviral zu behandeln.

Hierbei versteht sich, dass bei der Behandlung von durch resistente/multi-resistente HCM-Viren ausgelösten Infektionskrankheiten in der kombinatorischen Verwendung PFA als DNA-Polymerase-Hemmer eingesetzt wird.

Zaknun et al., "Concurrent Ganciclovir and Foscarnet treatment for cytomegalovirus encephalitis and retinitis in an infant with acquired immunodeficiency syndrome: case report and review", Pediatr. Infect. Dis. J. 1997, 16:807-811, berichten zwar von einer klinischen Besserung der HCMV-Infektion bei einer bestehenden HIV-Infektion nach einer Heranziehung von PFA unter Therapie mit GCV, AZT und ddI, jedoch wurde hier der synergetische Effekt von GCV und PFA als entscheidender Faktor gewertet. Einen Hinweis, dass gerade die Verwendung von RTI und PFA die Virus-hemmende Wirkung erzielte, ist in dieser Veröffentlichung nicht zu finden und auch nicht nahegelegt, da eine Wirkung von RTI in Kombination mit DNA-Polymerase-Hemmern gegenüber DNA-Viren nach Erkenntnis der Wissenschaft bisher nicht für möglich gehalten wurde.

Die Erfinder zeigten weiterhin, dass bereits geringe Mengen an RTI und DNA-Polymerase-Hemmer ausreichten, um nachhaltige virostatische Effekte zu erzielen.

Durch die Dosisreduktion der Wirkstoffkombinationen gegenüber den Einzelsubstanzen können toxische Begleiteffekte reduziert werden, also insbesondere die Nephrotoxizität der DNA-Polymerase-Hemmer.

Ein weiterer Vorteil der Kombinations-Therapie einer GCV-resistenten HCMV-Infektion liegt darin, dass die Erfindung sofort klinisch umgesetzt werden kann, da die Einzelkomponenten bereits in die jeweilige antivirale Therapie von HIV und/oder HCMV eingeführt sind.

Das die Kombinationen enthaltene Arzneimittel kann dabei entweder parenteral und/oder oral verabreicht werden.

Ferner können die Kombinationen zusammen mit einem oder mehreren pharmazeutisch akzeptablen Trägern verabreicht werden.

Unter "Trägern" oder Hilfsstoffen werden hierbei solche Substanzen verstanden, die in der Arzneimittelherstellung üblicherweise als Verdünnungsmittel, Bindemittel, Suspensionsmittel, Schmiermittel, Stabilisatoren usw. entweder als Lösung oder als feste Substanz verwendet werden. Dazu zählen, sind aber nicht hierauf beschränkt, beispielsweise Wasser, Salzlösungen, Alkohole, pflanzliche Öle, Polyethylenglucole, Gelatine, Laktose, Glucose, Amylose, Zellulose, Glycerol, Magnesiumstearat, Albumin, Monoglyceride, Diglyceride, Polydinylpyrolidon, etc. Eine Reihe von weiteren geeigneten Substanzen findet sich in A. Kibbe, Handbook of Pharmaceutical Excipients, 3. ed., 2000, American Pharmaceutical Association and Pharmaceutical Press.

Die pharmazeutische Zubereitung kann, wenn erwünscht, sterilisiert werden und kann außerdem Konservierungsmittel, Stabilisierungs- und/oder Dispersionsmitel, Emulgatoren, Puffer, Farbstoffe, Geschmacksstoffe, etc. enthalten, die mit der aktiven Verbindung nicht interferieren.

Orale Verabreichungen können in Form von beispielsweise Tabletten, Kapseln, Pastillen oder als flüssige Zubereitungen in Form eines Sirups vorliegen, die nach bekannten Verfahren, beispielsweise unter Zusatz von Kohlenhydrat-Bindemitteln wie Maisstärke etc., hergestellt werden. Für eine parenterale Verabreichung kann die Substanz als Injektion oder Infusion ebenfalls nach bekannten Verfahren formuliert werden.

Der Wirkstoff kann dabei auch beispielsweise in Liposomen und/oder Nanopartikel eingebaut werden, wobei die jeweils eingeführten lipophilen Reste die Größe und Stabilität der Liposomen maßgeblich beeinflussen.

Der Wirkstoff kann darüber hinaus auch in Verbindung mit anderen Wirkstoffen oder Arzneimitteln bei einer therapeutischen Anwendung eingesetzt werden, wie beispielsweise Reverse-Transkriptase-Hemmer, Protease-Hemmer, Interferon oder Interleukin II, oder bei immunmodulierenden Therapien wie Knochenmarks-oder Stammzelltransplantationen/Transplantationen solider Organe, oder mit Wirkstoffen, die die Anzahl der Lymphocyten steigern. Es können auch gleichzeitig mehrere Nukleosid-Phosphonoameisensäure-Derivate in einer Formulierung eingesetzt werden.

Eine genaue Dosierung hängt dabei vom Verabreichungsweg, dem zu behandelnden Zustand, der Schwere der Krankheit, dem Gewicht und dem Alter des zu behandelnden Patienten, etc. ab.

Es versteht sich, dass die vorstehend beschriebenen und nachstehend noch zu erläuternden Merkmale nicht nur in der jeweils angegebenen Kombination, sondern auch in anderen Kombinationen oder auch in Alleinstellung verwendbar sind, ohne den Rahmen der vorliegenden Erfindung zu verlassen.

Weitere Vorteile ergeben sich aus dem Beispiel.

### Beispiel

Zur antiviralen Testung wurde ein zelladaptierter Plaque-Reduktionsassay eingesetzt (siehe Prix et al., "A Simplified Assay for Screening of Drug Resistance of Cell-Associated Cytomegalovirus Strains", J. Clin. Virol. 1998; 11: 29 - 37). Beispielhaft wurden dabei folgende Virusstämme eingesetzt:
"Isolat 3": GCV-resistenter HCMV-Stamm, Mutation im UL 97-Gen Cys603Trp aus dem Ringversuch "GCV-Resistenz 2000", Gesellschaft für Virologie;
GCV-ID₅₀ (µM): 20,1
PFA-ID₅₀ (µM): 154
"MM159838": HCMV-Stamm aus Muttermilch einer Transmitterin vom Tag 31 nach der Geburt. Wildtypstamm, UL97-Wildtypsequenz (Cys603);
GCV-ID₅₀ (µM): < 6 (per definitionem)
PFA-ID₅₀ (µM): 74,2 - 201,4; < 400 (per definitionem)

Beispielhaft wurden Kombinationen aus NRTI in Verbindung mit dem DNA-Polymerase-Hemmer PFA unter Verwendung folgender Virostatika-Endkonzentrationen eingesetzt:
PFA: Messbereich 100 µM bis 1000 µM
AZT/3TC/ddC/d4T/ddI: 2,5 µM bis 50 µM

### Zelladaptierter Plague-Reduktionsassay

Um die antivirale Aktivität verschiedener Kombinationen von RTI und DNA-Polymerase-Hemmern nachzuweisen, wurde ein modifizierter zelladaptierter Plaque-Reduktionsassay durchgeführt. Dafür eingesetzt wurden Kokulturen von infizierten und nicht infizierten humanen Vorhautfibroblasten (HFF).

In Prix et al., A simplified assay for screening of drug resistance of cell-associated cytomegalovirus strains, Journal of Clinical Virology 1998, 11: 29-37, und Prix et al., Comprehensive restriction analysis of the UL97 region allows early detection of ganciclovir-resistant human cytomegalovirus in an immunocompromised child, Journal of infectious deseases 1999, 180: 491-495, ist die Durchführung des Assays zu ersten Mal beschrieben und wird nachstehend kurz dargestellt.

### Vorbereitung der HFF-Zellen

HCMV-Primärisolate auf HFF-Zellen in Röhrchenkultur wurden auf kleine Zellkulturflaschen (25 cm²) mit dichtem HFF-Zellrasen umgesetzt und kultiviert. Nach Erreichen eines ca. 20 - 40%igen zytopathischen Effekts (CPE) wurden die Zellen mit Phosphatgepufferter Kochsalzlösung (PBS) gewaschen und der Zellrasen abtrypsiniert. Nach Trypsininaktivierung wurde das Pellet in Eagles minimalem essentiellen Medium (MEM)-10% FCS (Fetales Kälberserum) aufgenommen und zur Trennung von Zellaggregaten schonend filtriert.

Parallel wurden die nicht infizierten HFF-Zellen auf 175 cm²-Schalen kultiviert, gewaschen, abtrypsiniert und nach weiteren Reinigungsschritten in MEM-10% FCS aufgenommen.

### Herstellung der Kokulturen

Zur Herstellung von Kokulturen wurden die infizierten Zellen mit den nicht infizierten folgendermaßen vermischt: 2,5 x 10⁴ nicht infizierte Zellen/100 µl wurden in vier unterschiedlichen Verhältnissen, mit 500, 1000, 2500 und 5000 infizierten Zellen /100 µl gemischt.

Von jeder dieser Verdünnungsstufen wurden jeweils 100 µl in einer Dreifachbestimmung auf eine 96-well Mitkrotiterplatte aufgetragen. Zur Zelladhärenz an den Mikrotiterplattenboden wurden die Kokulturen mindestens 6 h inkubiert, bevor das Medium vorsichtig entfernt wurde.

### Auftragen der Virostatika

Nach 1 Stunde Inkubation bei 37°C wurden 100 µl der entsprechenden Virostatikaverdünnungen (Probeansätze) oder Kulturmedium zugesetzt. Jeweils 24 Mikrokulturen wurden ohne Virostatika mitgeführt (Virus-Kontrollen für Referenzwert 100 % Plaquebildung). Ferner wurden mindestens drei Mikrokulturreplika von PFA und dem jeweiligen NRTI (also jeweils als getrennte vorliegende Einzelsubstanzen) neben drei Replika der Cokultur von PFA + NRTI mitgeführt (Toxizitätskontrolle).

Anschließend wurden die Kulturen bis sechs Tage inkubiert, so dass sich die Viren von den infizierten Einzelzellen in Form von Plaquebildung durch Infektion der Nachbarzellen ausbreiten konnten.

### HCMV-Nachweis mittels in situ-ELISA

Im Anschluss an die Inkubationszeit wurde das Medium schonend abgegossen und die Mikrokulturen mit 200 µl eiskaltem (-20°C) Methanol permeabilisiert und fixiert. Nach 20 min wurde das Methanol entfernt und die Zellen zweimal mit PBS gewaschen.

Anschließend wurde der primäre Antikörper (anti-IE1-pp72, E13 Klon, Paesel & Lorei, Deutschland) aufgetragen, der gegen das HCMV Immediate Early Antigen (UL123) von HCMV gerichtet ist. Nach 1 h Inkubation wurden die Zellen gewaschen und mit dem sekundären Antikörper (Meerrettich-Peroxidase konjugierter Maus-HCMV-IgG-Antikörper; DAKO, Dänemark) für 1 h inkubiert. Nach Zugabe des Peroxidase/H₂O₂-substrats wurde durch die in situ Braunfärbung das Immediate Early Antigen von HCMV in den Kernen infizierter Fibroblasten unter dem Mikroskop nachgewiesen. Das Ergebnis wurde in ausgewählten Beispielen fotographisch dokumentiert.

Im mikroskopischen Bild stellten sich infizierte Fibroblastenkerne braun dar. Nur die Plaquebildung reflektiert die vollständige Virusreplikation in allen drei Phasen (immediate early, early, late). Einzeln gefärbte Kerne nach fünftätiger *in vitro* Kultur reflektieren abortive Infektionen (Virusexpression blieb auf die IE-Stufe beschränkt).

Für eine korrekte Auswertung galt, dass ein Plaque aus einem Zellverband von mindestens 10 Einzelzellen bestehen musste, um in der Zählung berücksichtigt zu werden. Ferner wurden nur die Verdünnungsstufen berücksichtigt, die in der Kontrollreihe ohne Virostatikum zwischen 15 und 60 Plaques verfügten. Die statistische Auswertung erfolgt durch nicht lineare Regressionsanalyse mittels der Probit-Analyse an Hand der SPSS-Software (Statistical Software Package, SPSS, München). Dabei wurde die Konzentration der Verbindung bestimmt, die notwendig ist, um die Anzahl der Plaques um 50 % zu reduzieren (Inhibitorische Dosis 50 %, ID₅₀-Werte).

### Ergebnisse:

### Erster Versuchsansatz zum Vergleich

Die Primärdaten zum Plaquereduktionsassay unter Verwendung von AZT und PFA sind in der nachfolgenden Tabelle 1 für den Virusstamm MM159838 (GCV-sensitiver HCMV-Stamm) wiedergegeben.

**Tabelle 1: GCV-sensitiver HCMV-Stamm aus Muttermilch**

| | | **AZT** | | | **PFA** | | | **AZT+PFA** | |
|---|---|---|---|---|---|---|---|---|---|
| | 1 | 2 | 3 | 4 | 5 | 6 | 7 | 8 | 9 |
| A | K | K | K | K | K | K | K | K | K |
| | 83 | 83 | 81 | 85 | 75 | 85 | 104 | 90 | 72 |
| B | 50 µM | 50 µM | 50 µM | 1000 µM | 1000 µM | 1000 µM | 50 µM + 1000 µM | 50 µM + 1000 µM | 50 µM + 1000 µM |
| MW | 49 | 46 | 44 | 14 | 16 | 14 | 2 | 1 | 0 |
| | | 46.3 | | | 14.7 | | | 1 | |
| C | 25 µM | 25 µM | 25 µM | 500 µM | 500 µM | 500 µM | 25 µM + 500 µM | 25 µM + 500 µM | 25 µM + 500 µM |
| MW | 68 | 57 | 59 | 16 | 19 | 14 | 4 | 4 | 3 |
| | | 61.3 | | | 16.3 | | | 3.7 | |
| D | 12.5 µM | 12.5 µM | 12.5 µM | 400 µM | 400 µM | 400 µM | 12.5 µM +400 µM | 12.5 µM + 400 µM | 12.5 µM + 400 µM |
| MW | 60 | 61 | 61 | 20 | 21 | 14 | 5 | 6 | 8 |
| | | 60.7 | | | 18.3 | | | 6.3 | |
| E | 10 µM | 10 µM | 10 µM | 300 µM | 300 µM | 300 µM | 10 µM + 300 µM | 10 µM + 300 µM | 10 µM + 300 µM |
| MW | 59 | 67 | 68 | 24 | 20 | 17 | 11 | 8 | 8 |
| | | 64.7 | | | 20.3 | | | 9 | |
| F | 5 µM | 5 µM | 5 µM | 200 µM | 200 µM | 200 µM | 5 µM + 200 µM | 5 µM + 200 µM | 5 µM + 200 µM |
| MW | 63 | 72 | 60 | 25 | 26 | 23 | 8 | 13 | 9 |
| | | 65.0 | | | 24.7 | | | 10.0 | |
| G | 2.5 µM | 2.5 µM | 2.5 µM | 100 µM | 100 µM | 100 µM | 2.5 µM +100 µM | 2.5 µM + 100 µM | 2.5 µM + 100 µM |
| MW | 85 | 78 | 85 | 43 | 43 | 45 | 29 | 27 | 33 |
| | | 82.7 | | | 43.7 | | | 29.9 | |
| H | K | K | K | K | K | K | K | K | K |
| | naw | 78 | 76 | 86 | 84 | 91 | 98 | 79 | 95 |
| | 85.7 | (n=23) | | | | | | | |

In der Tabelle zeigen die linken drei Spalten Messungen mit der Substanz AZT in unterschiedlichen Konzentrationen (50 µM bis 2.5 µM) alleine, die Spalten 4 bis 6 Messungen mit der Substanz PFA (1000 µM bis 100 µM) in unterschiedlichen Endkonzentrationen alleine, und in den Spalten 7 bis 9 sind die Messungen für die Kombination der beiden Substanzen in unterschiedlichen Endkonzentrationen wiedergegeben. In der Tabelle ist die Anzahl der Plaques für die einzelnen Versuchsansätze (jeweils 3 Ansätze/Test) gezeigt, wobei die jeweiligen Mittelwerte (MW) für die 3 Versuchsansätze jeweils in der zweiten Spalte unter dem ermittelten Wert zu finden sind (also für AZT alleine in Spalte 2 (B2 - G2), für PFA alleine in Spalte 5 (B5 - G5), für die Kombination in Spalte 8 (B8 - G8)). Über der Anzahl der ermittelten Plaques ist jeweils die eingesetzte Endkonzentration (µM) der Substanzen im Test angegeben. In den Zeilen A und H sind ferner jeweils die Kontrollen wiedergegeben (keine Virostatika, Mittelwert H1). Der verwendete Virusstamm war MM159838 (siehe oben).

Wie der Tabelle zu entnehmen ist, zeigt die Kombination von AZT mit PFA eine deutlich bessere Reduzierung der Plaques als die Einzelsubstanzen alleine. So konnte beispielsweise bei einem Einsatz von 12,5 µM AZT in Gegenwart von 400 µM PFA (siehe jeweils D7, D8 und D9) die Anzahl der Plaques auf durchschnittlich 6,3 reduziert werden im Vergleich zu der durchschnittlichen Plaquezahl von 60,7 bei einem Einsatz der gleichen Menge AZT alleine und von einer durchschnittlichen Plaquezahl von 18,3 bei der gleichen Menge PFA alleine. Die ID₅₀ für AZT alleine betrug 67.0 µM (95% Konfidenzintervall, CI: 27 - 24574), für PFA alleine 74.2 µM (CI: 29.8 - 116.1). In der Kombination betrug die ID₅₀ für AZT 1.0 µM (CI: 0.4 - 1.7), für PFA 56.2 µM (CI: 29.9 - 80.8).

Dies zeigt eindrucksvoll die synergistische Wirkung der Substanzen, so dass es hiernach auch möglich ist, - im Vergleich zu einem Einsatz der Einzelsubstanzen - die einzusetzenden Mengen an Virostatika deutlich zu reduzieren. In vivo können die einzusetzenden Mengen mit Rücksicht auf Größe und Gewicht des Patienten sowie Schwere der Erkrankung jeweils auf den Patienten abgestimmt werden. In jedem Fall können aber bei der Kombination der Substanzen jeweils geringere Mengen eingesetzt werden als dies bei einer Verwendung der Einzelsubstanzen möglich wäre.

In weiteren Versuchen wurde der *in vitro*-Synergismus der Kombination von AZT und PFA auch gegenüber einem GCV-resistenten HCMV-Stamm ("Isolat 3", siehe oben) getestet. Ermittelt wurden die ID₅₀-Werte (µM) sowie das 95 % Konfidenzintervall (CI) in drei unabhängigen Versuchen. Die Ergebnisse dieser Tests sind in der nachstehenden Tabelle 2 aufgeführt:

**Tabelle 2: GCV-resistenter HCMV-Stamm**

| **ID₅₀ (µM)** | | | | | | | |
|---|---|---|---|---|---|---|---|
| **Einzelkomponente (µM)** | | | | **Wirkstoffkombination (µM)** | | | |
| 2.5-50 | | 100-1000 | | 100-1000 | | 2.5-50 | |
| **AZT** | CI | **PFA** | CI | **AZT+PFA** | CI | **AZT+PFA** | CI |
| **59.3** | 32.5-241.0 | **126.7** | 80.1-173.5 | **88.1** | 45.8-124.2 | **1.8** | 0.7-3.0 |
| **166.0** | 64.5-2567.1 | **160.0** | 127-195.0 | **73.6** | 35-108.4 | **1.4** | 0.5-2.4 |
| **94.9** | 62.2-221.7 | **114.2** | 90-135.5 | **106.4** | 80.3-129 | **2.5** | 1.7-3.2 |

Wie der Tabelle zu entnehmen ist, war der ID₅₀-Wert für AZT in der Kombination (AZT+PFA) deutlich niedriger als bei AZT als Einzelsubstanz. Daneben zeigte sich, dass mit der Kombination aus NRTI und DNA-Polymerase-Hemmer sogar auf Ganciclovir-resistente HCMV-Stämme eine virus-inhibierende Wirkung erzielt werden kann.

Der Wirksteigungsindex, der folgendermaßen berechnet wurde: SI_{AZT}= ID₅₀AZT / ID₅₀(AZT+PFA), betrug für AZT 32.9, 118.9, 38.0 bezogen auf die drei unabhängigen Versuche. Dieser Index beschreibt den Faktor, um den die virostatische Wirkung der Einzelsubstanz in der Kombination gesteigert wurde.

In einem zweiten Versuchsansatz wurde die Substanz d4T mit PFA kombiniert. Die Versuche wurden wie oben ausgeführt vorgenommen. Die Primärdaten des Plaquereduktionassays sind in der folgenden Tabelle 3 wiedergegeben:

**Tabelle 3: GCV-sensitiver HCMV-Stamm aus Muttermilch**

| | | **d4T** | | | **PFA** | | | **d4T+PFA** | |
|---|---|---|---|---|---|---|---|---|---|
| | 1 | 2 | 3 | 4 | 5 | 6 | 7 | 8 | 9 |
| A | K | K | K | K | K | K | K | K | K |
| | 80 | 79 | 82 | 74 | 78 | 82 | 78 | 74 | 84 |
| B | 50 µM | 50 µM | 50 µM | 1000 µM | 1000 µM | 1000 µM | 50µM+ 1000µM | 50µM+ 1000µM | 50µM+ 1000µM |
| | 77 | 76 | 74 | 11 | 9 | 11 | 1 | 0 | 0 |
| MW | | 75.7 | | | 10.3 | | | 0.3 | |
| C | 25 µM | 25 µM | 25 µM | 500 µM | 500 µM | 500 µM | 25µM+ 500µM | 25µM+ 500µM | 25µM+ 500µM |
| | 72 | 68 | 81 | 12 | 15 | 21 | 7 | 4 | 3 |
| MW | | 73.7 | | | 16.0 | | | 4.7 | |
| D | 12.5 µM | 12.5 µM | 12.5 µM | 400 µM | 400 µM | 400 µM | 12.5µM+ 400µM | 12.5µM+ 400µM | 12.5µM+ 400µM |
| | 69 | 75 | 82 | 10 | 16 | 13 | 6 | 6 | 7 |
| MW | | 75.3 | | | 13.0 | | | 6.3 | |
| E | 10 µM | 10 µM | 10 µM | 300 µM | 300 µM | 300 µM | 10µM+ 300µM | 10µM + 300µM | 10µM + 300µM |
| | 88 | 88 | 72 | 16 | 20 | 20 | 13 | 9 | 14 |
| MW | | 82.7 | | | 18.7 | | | 12.0 | |
| F | 5 µM | 5 µM | 5 µM | 200 µM | 200 µM | 200 µM | 5µM + 200µM | 5µM + 200µM | 5µM + 200µM |
| | 80 | 76 | 84 | 25 | 27 | 34 | 30 | 30 | 22 |
| MW | | 80 | | | 28.7 | | | 27.3 | |
| G | 2.5 µM | 2.5 µM | 2.5 µM | 100 µM | 100 µM | 100 µM | 2.5µM+ 100µM | 2.5µM+ 100µM | 2.5µM+ 100µM |
| | 79 | 88 | 66 | 48 | 38 | 40 | 38 | 41 | 38 |
| MW | | 77.7 | | | 42.0 | | | 39.0 | |
| H | K | K | K | K | K | K | K | K | K |
| | Naw | | | | | | | | |
| | | 102 | 79 | 85 | 87 | 79 | 87 | 100 | 75 |
| MW | 82.3 | n = 21 | | | | | | | |

Der verwendete Virusstamm war auch hier - wie in den Tests mit AZT - der Stamm MM159838, ein GCV-sensitiver HCMV-Stamm aus Muttermilch. In der Tabelle zeigen die linken drei Spalten Messungen mit der Substanz d4T in unterschiedlichen Endkonzentrationen (50 µM bis 2.5 µM) alleine, die Spalten 4 bis 6 Messungen mit der Substanz PFA in unterschiedlichen Endkonzentrationen (1000 µM bis 100 µM) alleine, und in den Spalten 7 bis 9 sind die Messungen für die Kombinationen der beiden Substanzen in unterschiedlichen Endkonzentrationen wiedergegeben. Auch diesen Daten ist zu entnehmen, dass die Kombination der beiden Substanzen gegenüber den Einzelsubstanzen eine deutlich verbesserte Plaquereduktion bewirkte. So ist beispielsweise bei einer Verwendung von 12,5 µM d4T mit 400 µM PFA eine Reduzierung der Plaques auf durchschnittlich 6,3 zu beobachten, wohingegen die Substanz d4T bei derselben Konzentration die Plaques lediglich auf durchschnittlich 75,3 reduzierte und die Substanz PFA bei einer Konzentration von 400 µM auf durchschnittlich 13,0.
Die ID₅₀ betrug in diesem Versuchsansatz für d4T alleine » 50 µM, für PFA 92.5 µM (CI: 51.1 - 129.8). In der Kombination betrug die ID₅₀ für d4T 2.5 µM (CI: 1.7 - 3.2), für PFA 105.4 µM (CI: 79.9 - 128.0).

Der *in vitro*-Synergismus wurde in weiteren Versuchen auch für die Substanzenkombinationen d4T/PFA, 3TC/PFA, ddC/PFA und als Vergleich ddI/PFA gegenüber einem GCV-resistenten HCMV-Stamm ("Isolat 3", siehe oben) und einem GCV-sensitiven HCMV-Stamm getestet. Die ermittelten ID₅₀-Werte (µM) sind in der nachstehenden Tabelle 4 wiedergegeben.

In der Tabelle bedeutet ">>50", dass keine nachweisbare Plaquereduktion gemessen werden konnte, und "Tox", dass bei 2.5 µM keine messbare Plaquereduktion vorlag, die Substanz aber ab 5 µM toxisch war.

**Tabelle 4:**

| **ID₅₀ (µM)** | | | | | | | |
|---|---|---|---|---|---|---|---|
| **Virusstamm** | **Einzelkomponente (µM)** | | | **Wirkstoffkombination (µM)** | | | |
| | 2.5-50 | 100-1000 | | 100-1000 | | 2.5-50 | |
| | **d4T** | **PFA** | CI | **d4T+PFA** | CI | **d4T-PFA** | CI |
| Isolat 3 | >> 50 | 114.2 | 90.0-135.5 | 99.9 | 34.1-151.3 | 2.2 | 0.3-4.2 |
| MM159838 | >> 50 | 92.5 | 51.1-129.8 | 105.4 | 79.9-128 | 2.5 | 1.7-3.2 |

| | **3TC** | **PFA** | CI | **3TC+PFA** | CI | **3TC+PFA** | CI |
|---|---|---|---|---|---|---|---|
| Isolat 3 | >> 50 | 114.2 | 90.0-135.5 | 108.0 | 91.8-151.8 | 2.5 | 1.6-3.4 |
| MM159838 | >> 50 | 153.9 | 78.4-216.1 | 144.0 | 77.8-200 | 3.6 | 1.2-6.0 |

| | **ddC** | **PFA** | CI | **ddC+PFA** | CI | **ddC+PFA** | CI |
|---|---|---|---|---|---|---|---|
| Isolat 3 | 36.8 | 114.2 | 90.0-135.5 | 84.1 | 55.0-109.7 | 1.8 | 1.0-2.6 |
| MM159838 | >> 50 | 153.9 | 78.4-216.1 | 144.0 | 77.8-200 | 3.6 | 1.2-6.0 |

| | **ddI** | **PFA** | CI | **ddI+PFA** | CI | **ddI+PFA** | CI |
|---|---|---|---|---|---|---|---|
| Isolat 3 | Tox | 114.2 | 90.0-135.5 | 54.3 | 23.8-83.7 | 0.9 | 0.004-2.5 |
| MM159838 | >> 50 | 201.4 | 71.9-310 | 147.1 | 58.7-216.2 | 3.6 | 0.4-7 |

Alle NRTI-Substanzen zeigten in der Kombination mit PFA eine inhibierende Wirkung sowohl gegenüber dem GCV-sensitiven HCMV-Stamm als auch gegenüber dem GCV-resistenten HCMV-Stamm. Der Tabelle ist ferner zu entnehmen, dass der für die NRTI in der Kombination ermittelte ID₅₀-Wert deutlich niedriger ist als jeweils bei der Verwendung der Einzelsubstanz alleine.

Die Erfinder konnten demnach ausführlich belegen, dass unter Verwendung von Kombinationen aus ausgewählten RTI und DNA-Polymerase-Hemmern eine Virus-hemmende Wirkung gegenüber DNA-Viren erzielt werden konnte.

## Patentansprüche

1. Verwendung von zumindest einem Hemmer der reversen Transkriptase (RTI) zur Herstellung eines Arzneimittels zur Behandlung von Erkrankungen und/oder Infektionen, die durch das humane Cytomegalovirus hervorgerufen werden, wobei das Arzneimittel zusätzlich zumindest einen Hemmer der viralen DNA-Polymerase aufweist und der zumindest eine RTI und der zumindest eine DNA-Polymerase-Hemmer in Form von getrennten Verbindungen vorliegen, **dadurch gekennzeichnet, dass** als RTI eine Verbindung eingesetzt wird, die ausgewählt ist aus der Gruppe bestehend aus 2',3'Didehydro-2',3'-didesoxy-thymidin (d4T), (-)-β-L-3'-Thia-2',3'-didesoxycytidin (3TC) und 2',3'-Didesoxycytidin (ddC) und als Hemmer der viralen DNA-Polymerase Foscarnet (PFA) eingesetzt wird.

2. Verwendung nach Anspruch 1, **dadurch gekennzeichnet, dass** die Kombinationen zur Herstellung eines Arzneimittels zur Behandlung von Viruserkrankungen eingesetzt werden, die durch Ganciclovir-sensitive humane Cytomegalovirus-Stämme hervorgerufen werden.

3. Verwendung nach Anspruch 1, **dadurch gekennzeichnet, dass** die Kombinationen zur Herstellung eines Arzneimittels zur Behandlung von Viruserkrankungen eingesetzt werden, die durch Ganciclovir-resistente humane Cytomegalovirus-Stämme hervorgerufen werden.

4. Verwendung nach Anspruch 1, **dadurch gekennzeichnet, dass** die Kombinationen zur Herstellung eines Arzneimittels zur Behandlung von Viruserkrankungen eingesetzt werden, die durch multiresistente humane Cytomegalovirus-Stämme hervorgerufen werden.

## Claims

1. Use of at least one inhibitor of reverse transcriptase (RTI) for the manufacturing of a medicament for treating diseases and/or infections which are caused by the human cytomegalovirus, wherein the medicament further comprises at least one inhibitor of viral DNA polymerase, and wherein the at least one RTI and the at least one DNA polymerase inhibitor are present in the form of separate compounds, **characterized in that** as RTI a compound is employed which is selected from the group consisting of 2',3'-didehydro-2',3'-dideoxythymidine (d4T), (-)-β-L-3'-thia-2',3'-dideoxycytidine (3TC) and 2',3'-dideoxycytidine (ddC), and that as inhibitor of the viral DNA polymerase Foscarnet (PFA) is employed.

2. The use as claimed in claim 1, **characterized in that** the combinations are employed for the manufacturing a medicament for treating viral diseases which are caused by human ganciclovir-sensitive cytomegalovirus strains.

3. The use as claimed in claim 1, **characterized in that** the combinations are employed for the manufacturing a medicament for treating viral diseases which are caused by human ganciclovir-resistant cytomegalovirus strains.

4. The use as claimed in claim 1, **characterized in that** the combinations are employed for the manufacturing a medicament for treating viral diseases which are caused by human multiresistant cytomegalovirus strains.

## Revendications

1. Utilisation d'au moins un inhibiteur de la transcriptase inverse (RTI) pour fabriquer un médicament destiné au traitement de maladies et/ou infections provoquées par le cytomégalovirus humain, le médicament présentant en plus au moins un inhibiteur de l'ADN polymérase virale et ledit au moins un RTI et ledit au moins un inhibiteur d'ADN polymérase étant présents sous la forme de composés séparés, **caractérisée en ce que** l'on utilise comme RTI un composé choisi dans le groupe constitué par la 2',3'-didéshydro-2',3'-didésoxythymidine (d4T), la (-)-β-L-3'-thia-2',3'-didésoxycytidine (3TC) et la 2',3'-didésoxycytidine (ddC), et comme inhibiteur de l'ADN polymérase virale le foscarnet (PFA).

2. Utilisation selon la revendication 1, **caractérisée en ce que** les combinaisons sont utilisées pour fabriquer un médicament destiné au traitement de maladies virales provoquées par des souches de cytomégalovirus humain sensibles au ganciclovir.

3. Utilisation selon la revendication 1, **caractérisée en ce que** les combinaisons sont utilisées pour fabriquer un médicament destiné au traitement de maladies virales provoquées par des souches de cytomégalovirus humain résistantes au ganciclovir.

4. Utilisation selon la revendication 1, **caractérisée en ce que** les combinaisons sont utilisées pour fabriquer un médicament destiné au traitement de maladies virales provoquées par des souches de cytomégalovirus humain multirésistantes.
